# EUROPEAN PATENT SPECIFICATION

(11) **EP 4 456 827 B1**
(45) Date of publication and mention of the grant of the patent: **03.12.2025**
(21) Application number: 22844291.9
(22) Date of filing: 28.12.2022
(51) Int. Cl.: A61C 9/00, A61B 90/00

(54) **UNIVERSAL MODULAR SCANNING TEMPLATE FOR INTRAORAL SCANNING**
UNIVERSELLE MODULARE ABTASTSCHABLONE FÜR INTRAORALES SCANNEN
GABARIT DE BALAYAGE MODULAIRE UNIVERSEL POUR BALAYAGE INTRA-BUCCAL

(30) Priority: 29.12.2021 IT 202100032858
(43) Date of publication of application: 06.11.2024
(73) Proprietor: Lastruttura S.p.A., 21012 Cassano Magnago (VA) (IT)
(72) Inventor: CAMPANA, Adriano, 21020 Brunello (IT)
(74) Representative: Modiano, Micaela Nadia
(86) International application number: PCT/IB2022/062838
(87) International publication number: WO 2023/126850

(56) References cited:
- WO-A1-2016/139557
- WO-A1-2021/087549
- US-A1- 2013 273 492
- US-A1- 2017 202 646

## Description

The present invention relates to a modular scanning template for intraoral scanning that is useful and practical in the field of oral implantology and in particular in methods for acquiring intraoral impressions by means of an intraoral scanner (known in the field by the acronym IOS).

As is known, the intraoral scanner is a three-dimensional acquisition instrument used to collect information about the shape and dimensions of dental arches so as to obtain a 3D digital impression.

Currently, in order to acquire an intraoral impression by means of an intraoral scanner it is necessary to use reference elements known as "scanbodies" (or "scan abutments" or "scan bodies") or the like, essentially constituted by pins, which are fixed in the arch of the oral cavity to be acquired and more precisely to the dental implants whose spatial position needs to be detected. These reference elements allow to acquire implant coordinates, by virtue of the digital alignment of a reference mathematics that exactly replicates the shape of the scanbody by mathematically identifying the position and rotation of each engagement element of each fixture, while the arch and tissues are acquired simply by viewing them with the scanner's video camera.

While necessary, these reference elements of the known type (scanbodies and the like) are sometimes not sufficient to obtain a satisfactory intraoral scan, especially in the presence of completely edentulous lower arches with limited stable saddle areas, even more so, if with immediate loading, with open flaps and unstable saddle area; in these cases, the critical and difficult-to-execute part of the scan is constituted by the transitions from one scanbody to the next.

In fact, in the field of digital scanning, one of the most critical issues during the acquisition of intraoral impressions is the management of soft tissues.

US2017/202646 discloses a modular apparatus for installation of multiple dental prostheses being adapted to connect and join various cylindrical stumps protruding from the maxillary or mandibular bone of a patient.

Intraoral scanners work well in the presence of stable and unchangeable complex morphologies, but are unable to acquire when what is being viewed and acquired changes, making it impossible for the instrument to recognize where it was previously and how to link successive images.

Therefore, currently it is often necessary to forgo digital acquisition particularly of critical impressions, or in any case great specific skill on the part of the operator and a greater amount of time are necessary, while in any case facing the risk of losing accuracy.

One known solution used to obviate this problem is to produce handcrafted connections between the scanbodies, for example with elastic bands. However, this method is not very effective, because elastic bands are poorly characterized, very symmetrical, fine and change over time, and therefore do not guarantee stable and unchangeable reference points useful for increasing precision and speed of acquisition.

The invention is as defined in the appended claims.

The aim of the present invention is to provide a template for intraoral scanning that is capable of solving the problems and overcoming the limitations of the background art that have been described above.

Within this aim, an object of the present invention is to provide a template for intraoral scanning that allows to obtain a valid intraoral scan even in critical situations, such as arches in which the patient has lost all teeth and the dentist has inserted fixtures, converting the edentulisms into arches without a single tooth but with fixtures placed, and with limited stable saddle area.

Another object of the invention is to provide a template for intraoral scanning that allows to perform an intraoral scan more rapidly.

Another object of the invention is to provide a template for intraoral scanning that allows to obtain a more precise and reliable intraoral scan.

Not least object of the invention is to provide a template for intraoral scanning that can be used also together with the reference pins of the known type (scanbodies, scan abutments, scan bodies or any other reference pin of the known type) and can be applied to any type of oral cavity.

This aim and these and other objects that will become better apparent hereinafter are achieved by a modular template for intraoral scanning according to claim 1.

Further characteristics and advantages will become better apparent from the description of a preferred but not exclusive embodiment of a modular template for intraoral scanning, illustrated by way of non-limiting example with the aid of the accompanying drawings, wherein:
Figure 1 is a plan view of a modular template for intraoral scanning, according to the invention, in a configuration for use inside an oral cavity of a patient;

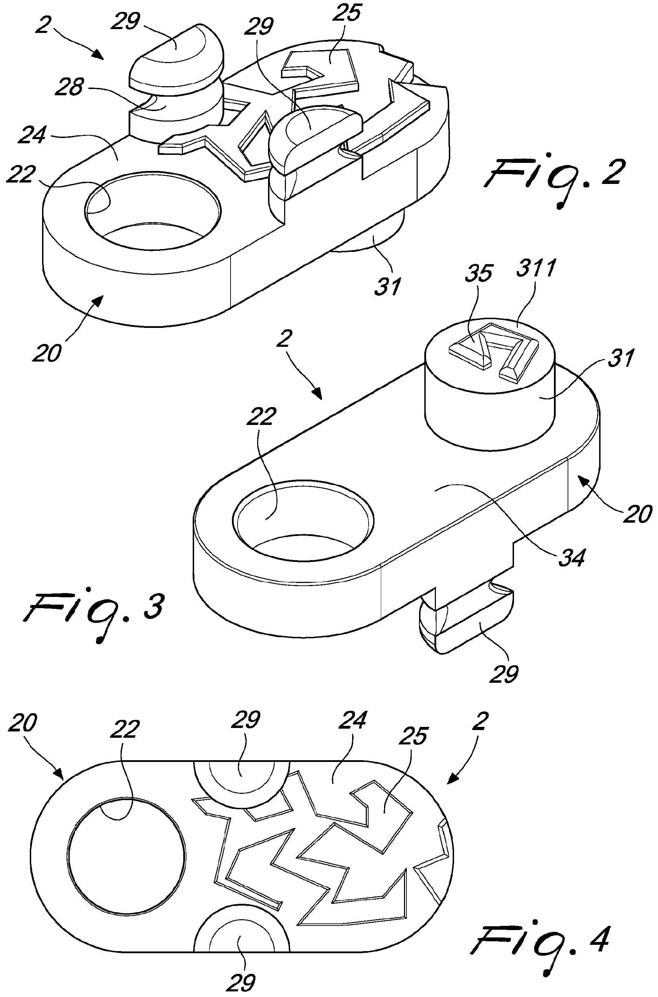

Figure 2 is a top perspective view of a reference element of the template of Figure 1;
Figure 3 is a bottom perspective view of the reference element of Figure 2;
Figure 4 is a top plan view of the reference element of Figure 2;

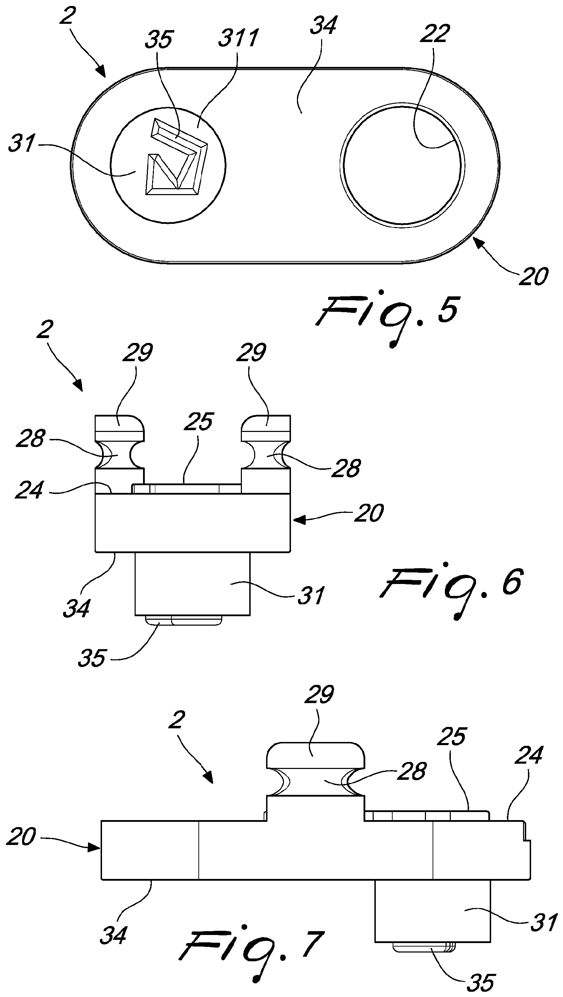

Figure 5 is a bottom plan view of the reference element of Figure 2;
Figures 6 and 7 are, respectively, a front elevation view and a lateral elevation view of the reference element of Figure 2;
Figure 8 is a view of a plurality of reference elements of the modular template, partially mutually assembled;
Figure 9 is a view of the reference elements of Figure 8, mutually assembled;
Figure 10 is a bottom perspective view of a variation of the element of the template of Figures 2 and 3.

With reference to the figures, the modular template for intraoral scanning, generally designated by the reference numeral 1, comprises a plurality of reference elements 2 adapted to be positioned in the oral cavity C of a patient in order to be detected by a scanner (i.e., by an acquisition device for acquiring digital images in order to obtain a digital impression of the oral cavity C).

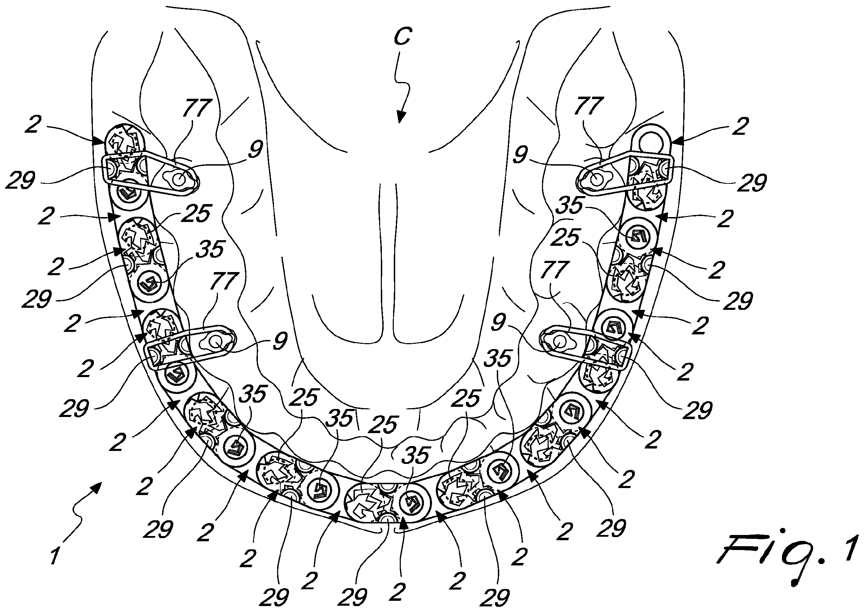

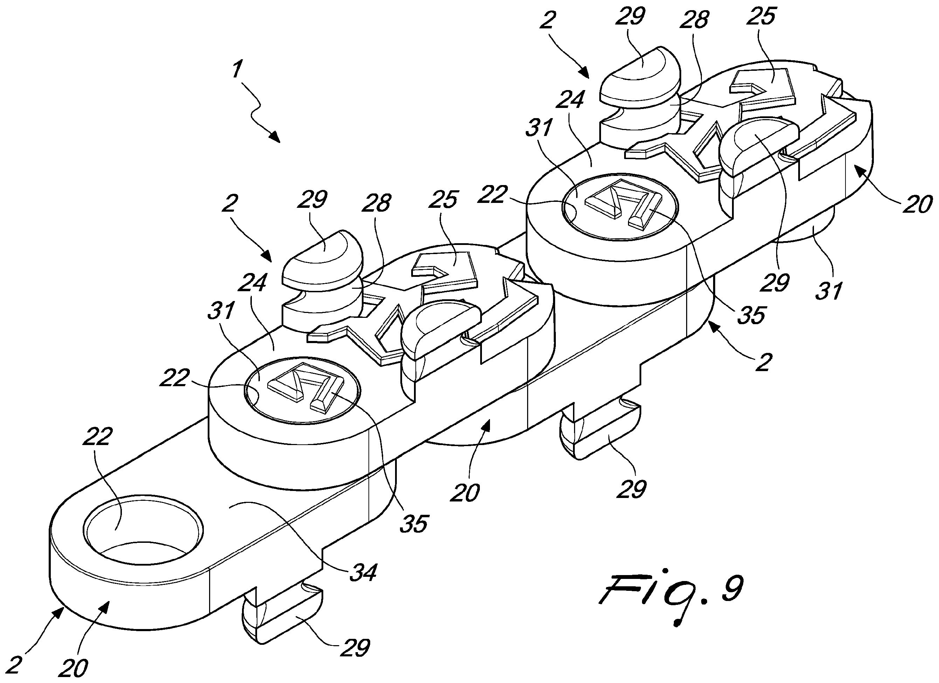

According to the invention, such reference elements 2 can be connected mechanically to each other in series, like a chain, for example as shown in Figure 1 and also in Figure 9.

In practice, each reference element 2 can be connected to a second reference element 2, which in turn can be connected to a third reference element 2, and so forth, until a chain (or, in other words, a template) of the length suitable for the purpose is formed.

The reference elements 2 are therefore parts that compose a chain or template and therefore the template 1 is modular in the sense that it can be composed by using any number of pieces (reference elements 2) according to the specific requirements.

As is evident from the figures, in the preferred embodiments all the reference elements 2 that compose the template are mutually identical, so as to be completely interchangeable.

In greater detail, each reference element 2 comprises a mechanical coupling element 31 (for example a pin or a hook) for coupling to another reference element 2.

In the preferred embodiment, the mechanical coupling element 31 comprises a pin which is configured to be inserted in a complementary hole 22 that is present in each reference element 2.

In the illustrated example, the mechanical coupling elements 31 consist of cylindrical pins adapted to be inserted in circular holes 22.

It should be noted that the pin that constitutes the mechanical coupling element 31 is arranged, in the individual reference element 2, at an opposite end with respect to the hole 22.

At least part of, and preferably all the, reference elements 2 comprise at least one fixing element 29 configured to be fixed to a reference pin 9 which can be fixed in the oral cavity C of the patient.

The reference pin 9 to which reference is made can be a reference pin of the known type commonly used in intraoral scanning, such as for example a scanbody (or scan abutment or scan body or the like).

Such fixing elements 29 consist preferably of vertically extended elements (with respect to the main body 20 of the reference element 2) which are adapted to be fastened by an elastic band 77 and/or are adapted to be glued to a respective reference pin 9.

In the preferred and illustrated embodiment, the fixing elements 29 are configured to be fixed by means of an elastic band 77 to a reference pin 9, as shown in Figure 1, and for this purpose are each provided with an external groove 28 adapted to be engaged by an elastic band 77; the same fixing elements 29 may optionally also be glued (for example, with a light-curing composite adhesive) to the reference pins 9, since they have an external wall that is adapted for this purpose.

In the preferred and illustrated embodiment, the fixing elements 29 have a substantially semi-cylindrical shape (i.e., the shape of a cylinder sectioned vertically in half), with the curved lateral wall directed toward the inside of the main body 20, and comprise a circumferential external groove 28 (on the curved lateral wall) and a flat external wall directed toward the outside of the main body 20.

Therefore, in practice, as shown in Figure 1, the template 1 can be configured in a configuration for use in which multiple reference elements 2 are mechanically connected to each other in series so as to form a chain or template which mutually connects a plurality of reference pins 9 (fixed in the oral cavity C) by means of some of the fixing elements 29, which are fixed to the reference pins 9 with elastic bands 77 or adhesive.

In even greater detail, in the preferred embodiments each reference element 2 comprises a main body 20 from which the mechanical coupling elements 31 and the fixing elements 29 protrude (at right angles).

This main body has two opposite flat faces 24, 34, a first face 24 and a second face 34, which are mutually parallel.

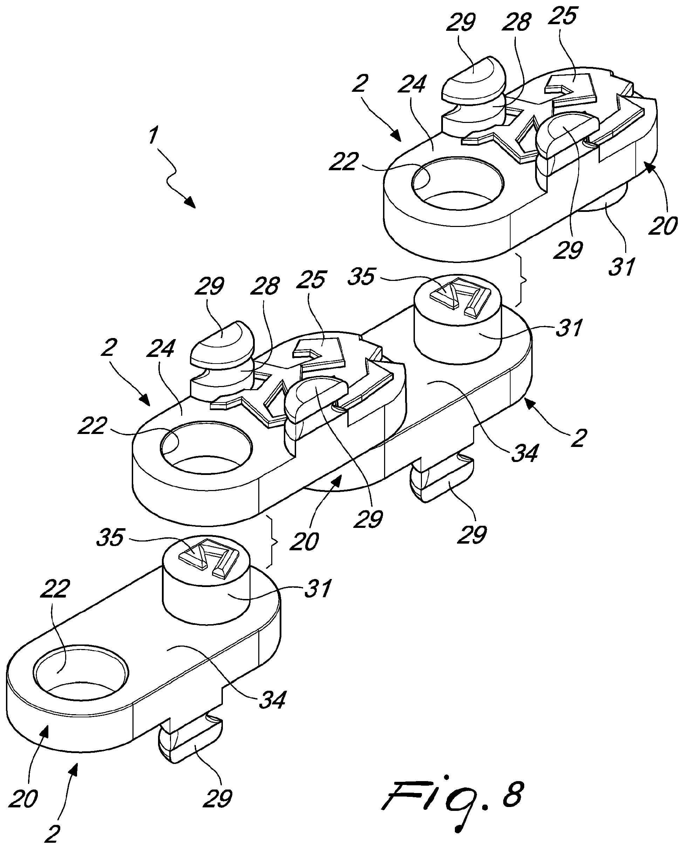

As can be seen in Figure 8, in the preferred embodiment the reference elements 2 are configured so that in said configuration for use they are oriented with the first face 24 directed in an alternating manner upward and downward, i.e., each one rotated through 180° with respect to the reference element to which is connected (a first reference element 2 arranged with the first face 24 directed downward is connected to a second reference element 2 whose first face 24 is directed upward, and said second reference element 2 is connected to a third reference element 2 whose first face 24 is directed downward, so that the first face 24 of the second reference element 2 rests partially on the second face 34 of the first reference element, and so forth).

Preferably, the main bodies 20 have, in plan view, a substantially oval or pseudo-rectangular shape comprising two flat lateral walls (at right angles to the first face 24 and the second face 34) connected by two curved sides.

Advantageously, the main bodies 20 have a rectangular profile in cross-section along a vertical plane.

Conveniently, the main bodies 20 have a flat extension and a flattened shape, in the sense that they extend along a plane and the height (in the direction at right angles to said plane) is less than the width and length.

In the preferred embodiments, including the one shown, the reference elements 2 each comprise two fixing elements 29 arranged on the first face 24 (protruding at right angles therefrom), at two opposite lateral ends of the main body 20.

The fact that in the preferred embodiment all the reference elements 2 comprise two fixing elements 29 configured to be able to be fixed to a reference pin 9 that can be fixed in the oral cavity C of the patient is particularly advantageous in terms of versatility: as can be deduced from Figure 1, all the fixing elements 29 are not necessarily used for this purpose, but this characteristic allows to fasten each reference pin 9 to the template, regardless of the specific spatial position within the oral cavity in which said pin 9 is located.

Preferably, the mechanical coupling element 31 is arranged on the second face 34 and protrudes at right angles therefrom.

According to a particularly advantageous characteristic, a shape protrudes from the first face 24 and forms a first graphic reference symbol 25. This graphic symbol 25 is adapted to be detected by the scanner and allows a better identification of the position and orientation of the reference element 2.

Such graphic symbol 25 is arranged so that it is free and visible even when, in the condition for use, the reference element 2 is connected to one or two other reference elements 2.

Preferably, this graphic symbol 25 comprises a perimeter constituted by an irregular polygonal broken line.

Optionally, the pin of the mechanical coupling element 31 comprises, on a free distal end 311 thereof, a protruding shape which forms a second graphic symbol 35, which preferably comprises a perimeter constituted by an irregular polygonal broken line. It should be noted from Figure 9 that when, in the configuration for use, the pin of a mechanical coupling element 31 of a reference element 2 is inserted in a through hole 22 of another reference element 2, the second graphic symbol 35 is visible and detectable by a scanner.

Conveniently, the reference elements 2 are made of biocompatible material.

The reference elements can be produced by 3D printing.

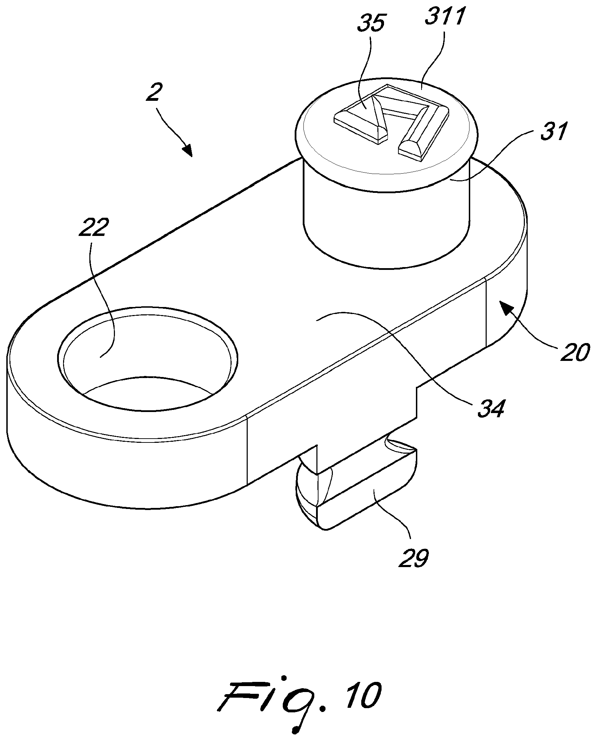

Conveniently, the reference element 2 can have the mechanical coupling element 31 provided with a top portion that protrudes with respect to the cylindrical body of the element 31, so as to define an undercut, as shown in Figure 10.

The operation of the template 1 is clear and evident from what has been described.

An optimum method for using the template 1 to obtain an intraoral scan comprises the following steps:
- fixing the reference pins 9 (scanbodies or the like) in the oral cavity C in a known manner, so that they mate perfectly with the fixtures and preferably verifying the quality of the coupling by control RX, if possible, or visually;
- assembling the reference elements 2, as shown in Figures 8 and 9, in order to obtain a single chain of the length appropriate for the case;
- resting the chain vestibularly on the reference pins 9 and bending it with the fingers until the correct arc, resting on all the reference pins 9, is obtained; the chain can be fixed to the reference pins 9 by using light-curing composite, which must be applied only to the wall of the pins 9 without ever invading the upper part of said pins; as an alternative, the chain can be fixed by using orthodontic elastic bands 77 (preferably medium elastic bands of the 1/8"- 3.2 mm type), which must be inserted on the body of the reference pin 9 and pulled on the adapted fixing elements 29 or wherever deemed convenient to hold the chain in place.

Once the chain has been fixed in the oral cavity C and fixed to the reference pins 9, it is possible to perform the scan of the oral cavity, which acquires images of the chain in position.

At the end of the acquisition, a scan of the reference pins 9, rendered mutually integral by virtue of the chain formed by the reference elements 2, is obtained. Thus, it is possible to perform the acquisition faster and in a more precise manner.

In practice it has been found that the modular template for intraoral scanning, according to the present invention, achieves the intended aim and objects, since it allows to obtain a valid intraoral scan even in critical situations such as on arches in which the patient has lost all the teeth and the dentist has inserted fixtures, converting the edentulisms into arches without a single tooth but with fixtures placed, and with little stable saddle area.

Another advantage of the modular template for intraoral scanning, according to the invention, resides in that it allows to perform the intraoral scan more rapidly.

An additional advantage of the modular template for intraoral scanning, according to the invention, resides in that it allows to perform a more precise and reliable intraoral scan.

Another advantage of the modular template for intraoral scanning, according to the invention, is that it is "universal" and can be applied to any type of reference pin (scanbody or scan abutment or scan body or any other reference pin of the known type) and to any type of oral cavity.

The modular template for intraoral scanning thus conceived is susceptible of numerous modifications and variations, all of which are within the scope of the accompanying claims.

All the details may furthermore be replaced with other technically equivalent elements.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A modular template (1) for intraoral scanning, comprising a plurality of reference elements (2) adapted to be positioned in the oral cavity (C) of a patient in order to be detected by a scanner,
**characterized in that** said reference elements (2) can be connected mechanically to each other in series, like a chain,
wherein each reference element (2) comprises a mechanical coupling element (31) for coupling to another reference element (2);
at least part of said reference elements (2) comprising a fixing element (29), wherein said fixing elements (29) consist of vertically extended elements which are adapted to be fastened by an elastic band (77) and/or are adapted to be glued to a respective reference pin (9) that is fixed in the oral cavity (C) of the patient.

2. The modular template according to claim 1, wherein said reference elements (2) comprise a first face (24) from which a shape protrudes which forms a first graphic reference symbol (25).

3. The modular template according to claim 1 or 2, wherein said mechanical coupling element (31) comprises a pin which is configured to be inserted in a complementary hole (22) that is present in each reference element (2).

4. The modular template according to claim 3, wherein the pin of the mechanical coupling element (31) comprises, on a free distal end (311) thereof, a protruding shape which forms a second graphic symbol (35).

5. The modular template according to one or more of the preceding claims, wherein each reference element (2) comprises a main body (20) from which said mechanical coupling elements (31) and said fixing elements (29) protrude.

6. The modular template according to the preceding claim, wherein said fixing elements (29) protrude at right angles from a first face (24) of the main body (20) of the reference element (2) and said mechanical coupling element (31) protrudes at right angles from a second face (34) of the main body (20) which is parallel and opposite with respect to said first face (24).

7. The modular template according to claim 6, wherein said reference elements (2) each comprise two of said fixing elements (29) arranged at two opposite lateral ends of the main body (20).

8. The modular template according to one of claims 5 to 7, wherein each main body (20) has, in plan view, a substantially oval or pseudo-rectangular shape which comprises two flat side walls connected by two curved sides.

9. The modular template according to claim 1, which can be configured in a configuration for use in which multiple reference elements (2) are mechanically connected to each other in series so as to form a chain which mutually connects a plurality of said reference pins (9) by means of some of said fixing elements (29), which are fixed to said reference pins (9) with elastic bands and/or adhesive.

## Patentansprüche

1. Modulare Schablone (1) für das intraorale Scannen, umfassend eine Vielzahl von Referenzelementen (2), die dazu ausgelegt sind, in der Mundhöhle (C) eines Patienten positioniert zu werden, um von einem Scanner erfasst zu werden,
**dadurch gekennzeichnet, dass** die Referenzelemente (2) mechanisch wie eine Kette in Reihe miteinander verbunden werden können,
wobei jedes Referenzelement (2) ein mechanisches Kupplungselement (31) zur Kopplung mit einem anderen Referenzelement (2) umfasst;
wobei zumindest ein Teil der Referenzelemente (2) ein Befestigungselement (29) umfasst, wobei die Befestigungselemente (29) aus vertikal verlängerten Elementen bestehen, die dazu ausgelegt sind, durch ein elastisches Band (77) befestigt und/oder an einen jeweiligen Referenzstift (9) geklebt zu werden, der in der Mundhöhle (C) des Patienten fixiert ist.

2. Modulare Schablone nach Anspruch 1, wobei die Referenzelemente (2) eine erste Fläche (24) umfassen, aus der eine Form hervorsteht, die ein erstes grafisches Referenzsymbol (25) bildet.

3. Modulare Schablone nach Anspruch 1 oder 2, wobei das mechanische Kupplungselement (31) einen Stift umfasst, der so konfiguriert ist, dass er in ein komplementäres Loch (22) eingeführt werden kann, das in jedem Referenzelement (2) vorhanden ist.

4. Modulare Schablone nach Anspruch 3, wobei der Stift des mechanischen Kupplungselements (31) an seinem freien distalen Ende (311) eine hervorstehende Form aufweist, die ein zweites grafisches Symbol (35) bildet.

5. Modulare Schablone nach einem oder mehreren der vorstehenden Ansprüche, wobei jedes Referenzelement (2) einen Hauptkörper (20) umfasst, aus dem die mechanischen Kupplungselemente (31) und die Befestigungselemente (29) herausragen.

6. Die modulare Schablone gemäß dem vorstehenden Anspruch, wobei die Befestigungselemente (29) rechtwinklig von einer ersten Fläche (24) des Hauptkörpers (20) des Referenzelements (2) hervorstehen und das mechanische Kupplungselement (31) rechtwinklig von einer zweiten Fläche (34) des Hauptkörpers (20) hervorsteht, die parallel und gegenüberliegend zu der ersten Fläche (24) ist.

7. Modulare Schablone nach Anspruch 6, wobei die Referenzelemente (2) jeweils zwei der Befestigungselemente (29) umfassen, die an zwei gegenüberliegenden seitlichen Enden des Hauptkörpers (20) angeordnet sind.

8. Modulare Schablone nach einem der Ansprüche 5 bis 7, wobei jeder Hauptkörper (20) in der Draufsicht eine im Wesentlichen ovale oder pseudo-rechteckige Form aufweist, die zwei flache Seitenwände umfasst, die durch zwei gekrümmte Seiten verbunden sind.

9. Modulare Schablone nach Anspruch 1, die in einer Konfiguration für die Verwendung konfiguriert werden kann, in der mehrere Referenzelemente (2) mechanisch in Reihe miteinander verbunden sind, um eine Kette zu bilden, die mehrere der Referenzstifte (9) mittels einiger der Befestigungselemente (29) miteinander verbindet, die mit Gummibändern und/oder Klebstoff an den Referenzstiften (9) befestigt sind.

## Revendications

1. Gabarit modulaire (1) pour balayage intra-oral, comprenant une pluralité d'éléments de référence (2) adaptés pour être positionnés dans la cavité buccale (C) d'un patient afin d'être détectés par un scanner,
**caractérisé en ce que** lesdits éléments de référence (2) peuvent être reliés mécaniquement les uns aux autres en série, comme une chaîne,
où chaque élément de référence (2) comprenant un élément de couplage mécanique (31) pour couplage à un autre élément de référence (2) ;
au moins une partie desdits éléments de référence (2) comprenant un élément de fixation (29), où lesdits éléments de fixation (29) consistent en des éléments s'étendant verticalement qui sont adaptés pour être fixés par un élastique (77) et/ou sont adaptés pour être collés à une broche de référence respective (9) qui est fixée dans la cavité buccale (C) du patient.

2. Le gabarit modulaire selon la revendication 1, où lesdits éléments de référence (2) comprennent une première face (24) à partir de laquelle fait saillie une forme qui forme un premier symbole de référence graphique (25).

3. Le gabarit modulaire selon la revendication 1 ou 2, où ledit élément d'accouplement mécanique (31) comprend une broche qui est configurée pour être insérée dans un trou complémentaire (22) qui est présent dans chaque élément de référence (2).

4. Gabarit modulaire selon la revendication 3, où la broche de l'élément d'accouplement mécanique (31) comprend, sur une extrémité distale libre (311) de celle-ci, une forme saillante qui forme un deuxième symbole graphique (35).

5. Le gabarit modulaire selon une ou plusieurs des revendications précédentes, où chaque élément de référence (2) comprend un corps principal (20) à partir duquel lesdits éléments d'accouplement mécaniques (31) et lesdits éléments de fixation (29) font saillie.

6. Le gabarit modulaire selon la revendication précédente, où lesdits éléments de fixation (29) font saillie à angle droit à partir d'une première face (24) du corps principal (20) de l'élément de référence (2) et ledit élément d'accouplement mécanique (31) fait saillie à angle droit à partir d'une deuxième face (34) du corps principal (20) qui est parallèle et opposée à ladite première face (24).

7. Gabarit modulaire selon la revendication 6, où lesdits éléments de référence (2) comprennent chacun deux desdits éléments de fixation (29) disposés aux deux extrémités latérales opposées du corps principal (20).

8. Gabarit modulaire selon l'une des revendications 5 à 7, où chaque corps principal (20) a, en vue en plan, une forme sensiblement ovale ou pseudo-rectangulaire qui comprend deux parois latérales plates reliées par deux côtés courbes.

9. Gabarit modulaire selon la revendication 1, qui peut être configuré dans une configuration d'utilisation dans laquelle plusieurs éléments de référence (2) sont reliés mécaniquement les uns aux autres en série de manière à former une chaîne qui relie mutuellement une pluralité desdites broches de référence (9) au moyen de certains desdits éléments de fixation (29), qui sont fixés auxdites broches de référence (9) à l'aide de bandes élastiques et/ou d'adhésif.
